# EUROPEAN PATENT SPECIFICATION

(11) **EP 4 389 144 B1**
(45) Date of publication and mention of the grant of the patent: **17.06.2026**
(21) Application number: 22871115.6
(22) Date of filing: 20.09.2022
(51) Int. Cl.: A61K 31/7048, A61K 31/7052, A61P 1/02, A61P 11/00, A61P 19/00, A61P 19/02, A61P 19/10, A61P 29/00, A61P 31/04

(54) **REGENERATIVE AGENT FOR BONE TISSUE, AND PHARMACEUTICAL COMPOSITION FOR REGENERATING BONE TISSUE**
REGENERATIONSMITTEL FÜR KNOCHENGEWEBE UND PHARMAZEUTISCHE ZUSAMMENSETZUNG ZUR REGENERATION VON KNOCHENGEWEBE
AGENT RÉGÉNÉRATEUR POUR TISSU OSSEUX, ET COMPOSITION PHARMACEUTIQUE POUR RÉGÉNÉRER UN TISSU OSSEUX

(30) Priority: 27.09.2021 JP 2021156321
(43) Date of publication of application: 26.06.2024
(73) Proprietor: Niigata University, Niigata-shi, Niigata 950-2181 (JP)
(72) Inventor: MAEKAWA, Tomoki, Niigata-shi, Niigata 951-8514 (JP); TABETA, Koichi, Niigata-shi, Niigata 951-8514 (JP); TERAO, Yutaka, Niigata-shi, Niigata 951-8514 (JP); TAMURA, Hikaru, Niigata-shi, Niigata 951-8514 (JP)
(74) Representative: Gill Jennings & Every LLP
(86) International application number: PCT/JP2022/034943
(87) International publication number: WO 2023/048125

(56) References cited:
- WO-A1-2006/137423
- WO-A1-2019/216438
- YUH DA-YO ET AL: "The secreted protein DEL-1 activates a [beta]3 integrin-FAK-ERK1/2-RUNX2 pathway and promotes osteogenic differentiation and bone regeneration", vol. 295, no. 21, 1 May 2020 (2020-05-01), US, pages 7261 - 7273, XP093215788, ISSN: 0021-9258, Retrieved from the Internet <URL:https://pdf.sciencedirectassets.com/778417/1-s2.0-S0021925817X50072/1-s2.0-S0021925817502616/main.pdf?X-Amz-Security-Token=IQoJb3JpZ2luX2VjEML//////////wEaCXVzLWVhc3QtMSJHMEUCIQDvRpF0cNhvLyskrWF9Yo3LkOaNcZYwOCPcBhHxSscVYwIgTznFZKBvAm0aMhLo2o++t0b1P/f0uGewsVYJqoMOsbkqsgUIKhAFGgwwNTkwMDM1NDY4NjUiDDS4/> DOI: 10.1074/jbc.RA120.013024
- TAMURA HIKARU ET AL: "Effects of Erythromycin on Osteoclasts and Bone Resorption via DEL-1 Induction in Mice", vol. 10, no. 3, 17 March 2021 (2021-03-17), pages 312, XP093214899, ISSN: 2079-6382, Retrieved from the Internet <URL:https://www.mdpi.com/2079-6382/10/3/312/pdf> DOI: 10.3390/antibiotics10030312
- TOMOKI MAEKAWA: "JR-2: Elucidation of anti-inflammatory mechanism of erythromycin via DEL-1", JOURNAL OF ORAL BIOSCIENCES, vol. 63, no. Suppl., 22 September 2021 (2021-09-22), pages 36, XP009544759, ISSN: 1349-0079
- TOMOKI MAEKAWA: "Bone metabolism control by DEL-1 molecular regulation and development to anti-aging", PRECISION MEDICINE, vol. 4, no. 5, 25 May 2021 (2021-05-25), pages 495 - 499, XP009544760, ISSN: 2434-3625
- MAEKAWA TOMOKI, TAMURA HIKARU, DOMON HISANORI, HIYOSHI TAKUMI, ISONO TOSHIHITO, YONEZAWA DAISUKE, HAYASHI NAOKI, TAKAHASHI NAOKI, : "Erythromycin inhibits neutrophilic inflammation and mucosal disease by upregulating DEL-1", JCI INSIGHT, vol. 5, no. 15, 6 August 2020 (2020-08-06), XP093052062, DOI: 10.1172/jci.insight.136706

## Description

### [Technical Field]

The present invention relates to a regenerative agent for bone tissue and lung tissue, and a pharmaceutical composition for regenerating bone tissue and lung tissue.

Priority is claimed on Japanese Patent Application No. 2021-156321, filed September 27, 2021.

### [Background Art]

Several bone regeneration therapies have been attempted for the living body's bone resorption and bone destruction associated with periodontitis and rheumatoid arthritis. For example, REGROTH (registered trademark) (manufactured by Kaken Pharmaceutical Co., Ltd.) containing basic fibroblast growth factor-2 (bFGF-2) as an active ingredient is the only drug approved as a bone regenerative agent in Japan.

Meanwhile, macrolide antimicrobial agents have been reported to have an anti-inflammatory action in addition to an antimicrobial action; however, the mechanism thereof is unknown. The inventors have hitherto reported that when a macrolide is administered to a living body, the macrolide promotes the production of DEL-1 molecules and exhibits an anti-inflammatory action and a bone resorption inhibitory action (see, for example, Non-Patent Document 1). In addition, it has been hitherto reported that the production induction pathway of DEL-1 is induced by resolvin D1 or DHEA (see, for example, Non Patent Documents 2 and 3), and it has been also made clear that the induction potency of erythromycin, which is a macrolide antimicrobial agent, is about three times the induction potency of those (see, for example, Non-Patent Document 1).

The inventors have also reported that DEL-1 induced in a living body contributes to the suppression of periodontitis (see, for example, Non-Patent Documents 1, 4, and 5) and pneumonia (see, for example, Non-Patent Document 1). Furthermore, it has been revealed that macrolide antimicrobial agents including erythromycin also directly suppress bone resorption. In addition, it has been also reported that DEL-1 induced by a macrolide antimicrobial agent is important for the proliferation of hematopoietic stem cells (see, for example, Non-Patent Document 6).

### [Citation List]

### [Non-Patent Documents]

[Non-Patent Document 1]
   Maekawa T et al., "Erythromycin inhibits neutrophilic inflammation and mucosal disease by upregulating DEL-1.", JCI Insight, Vol. 5, Issue 15, e136706, 2020.
[Non-Patent Document 2]
   Maekawa T et al., "Antagonistic effects of IL-17 and D-resolvins on endothelial Del-1 expression through a GSK-3β-C/EBPβ pathway.", Nature Communications, Vol. 6, Article number: 8272, DOI: 10.1038/ncomms9272, 2015.
[Non-Patent Document 3]
   Ziogas A et al., "DHEA Inhibits Leukocyte Recruitment through Regulation of the Integrin Antagonist DEL-1.", J Immunol, Vol. 204, Issue 5, pp. 1214-1224, 2020.
[Non-Patent Document 4]
   Eskan MA et al., "The leukocyte integrin antagonist Del-1 inhibits IL-17-mediated inflammatory bone loss.", Nature Immunology, Vol. 13, No. 5, pp. 465-473, 2012.
[Non-Patent Document 5]
   Shin J et al., "DEL-1 restrains osteoclastogenesis and inhibits inflammatory bone loss in nonhuman primates.", Science Translational Medicine, Vol. 7, issue 307, 307ra155, 2015.
[Non Patent Document 6]
   Mitroulis I et al., "Secreted protein Del-1 regulates myelopoiesis in the hematopoietic stem cell niche.", J Clin Invest, Vol. 127, No. 10, pp. 3624-3639, 2017.

### [Summary of Invention]

### [Technical Problem]

However, in the bone regeneration therapies reported so far, the amount of bone to be restored is not sufficient, and many of the therapies are not recognized to have effect on inflammations and bacterial infections.

The present invention has been made in consideration of the above circumstances and provides a novel regenerative agent for bone tissue and lung tissue, which can regenerate bone tissue or lung tissue.

### [Solution to Problem]

The inventors of the present invention conducted thorough studies in order to achieve the above-described object, and as a result, they found that a developmental endothelial locus-1 (DEL-1) induced by a macrolide antimicrobial agent induces proliferation and differentiation of mesenchymal stem cells and contributes to regeneration of bone and lung tissues, thereby completing the present invention.

That is, the present invention includes the following aspects. A macrolide antimicrobial agent for use in regenerating bone tissue, wherein the macrolide antimicrobial agent is azithromycin. In an embodiment, the macrolide antimicrobial agent is parenterally administered to bone tissue in the form of an injectable preparation, and wherein the dose **is 50** µg **to** 200 µg per day in an adult. In another embodiment the macrolide antimicrobial agent is administered orally, and the dose is 100 µg to 1200 mg per day in an adult.
A macrolide antimicrobial agent for use in treating a disease accompanied with the destruction of bone tissue, wherein the macrolide antimicrobial agent is azithromycin, and wherein the disease accompanied with the destruction of bone tissue is osteoporosis or rheumatoid arthritis.

### [Advantageous Effects of Invention]

According to the regenerative agent for bone tissue and lung tissue and the pharmaceutical composition for regenerating bone tissue and lung tissue according to the above-described aspect, bone tissue or lung tissue can be regenerated, and a disease accompanied with destruction of bone tissue or lung tissue can be effectively treated.

### [Brief Description of Drawings]

FIG. 1 is stained images of bone nodules generated by each osteoblast in Example 1.
FIG. 2 is a graph showing proportions of bone nodules in each osteoblast and relative expression levels of Runx2 mRNA, Sp mRNA, and Bglap mRNA in Example 1.
FIG. 3 is a graph showing the amount of bone regeneration in wild-type mice and DEL-1-deficient experimental periodontitis model mice to which erythromycin has or has not been administered in Example 2.
FIG. 4 is a graph showing the amount of bone regeneration in an aged experimental periodontitis model mouse to which erythromycin has or has not been administered in Example 2.
FIG. 5 is fluorescent immunostaining images of periodontal tissue of wild-type experimental periodontitis mice to which erythromycin has or has not been administered in Example 2.
FIG. 6 is captured microCT images of a hind limb of each of rheumatoid arthritis model mice in Example 3.
FIG. 7 are hematoxylin-and eosin-stained images of lung tissues of a wild-type mouse (A) in which acute lung disorder is induced without administration of erythromycin, a wild-type mouse (B) in which acute lung disorder is induced with administration of erythromycin, and a DEL-1-deficient mouse (C) in which acute lung disorder is induced with administration of erythromycin, in Example 4.
FIG. 8 is a graph showing relative expression levels of DEL-1 mRNA in each of vascular endothelial cells in Example 5.
FIG. 9 is a graph showing the amounts of bone regeneration in aged experimental periodontitis model mice to which a macrolide antimicrobial agent has or has not been administered in Example 5.
FIG. 10 is a graph showing the amounts of regeneration of jaw bones in young or aged cynomolgus monkeys to which a macrolide antimicrobial agent or REGROTH has or has not been administered in Example 6.
FIG. 11 is a graph showing the proportion of bone nodules in each osteoblast in Example 7.
FIG. 12 is stereomicroscopic images of teeth of aged mice to which a macrolide antimicrobial agent has or has not been administered in Example 8.
FIG. 13 is a graph showing the amounts of tooth regeneration in aged mice to which a macrolide antimicrobial agent has or has not been administered in Example 8.
FIG. 14 is a graph showing the numbers of DEL-1-positive cells in the jawbones of aged mice to which a macrolide antimicrobial agent has or has not been administered in Example 9.
FIG. 15 is a graph showing the numbers of α-SMA-positive cells in the jawbones of aged mice to which a macrolide antimicrobial agent has or has not been administered in Example 9.
FIG. 16 is immunostained images of aging cells in the jawbones of aged mice to which a macrolide antimicrobial agent has or has not been administered in Example 9.
FIG. 17 is a graph showing the numbers of aging cells in the jawbones of aged mice to which a macrolide antimicrobial agent has or has not been administered in Example 9.
FIG. 18 is stained images of bone nodules in bone cells differentiated from mesenchymal stem cells induced from human iPS cells under the conditions with or without the addition of a macrolide antimicrobial agent in Example 10.
FIG. 19 is a graph showing the proportions of bone nodules in bone cells differentiated from mesenchymal stem cells induced from human iPS cells under the conditions with or without the addition of a macrolide antimicrobial agent in Example 10.

### [Description of Embodiments]

Hereinafter, a regenerative agent for bone tissue and lung tissue and a pharmaceutical composition for regenerating bone tissue and lung tissue according to the disclosure will be described in detail.

### <Regenerative agent for bone tissue and lung tissue>

A regenerative agent for bone tissue and lung tissue of the present embodiment contains a macrolide antimicrobial agent as an active ingredient, wherein the macrolide antimicrobial agent is azithromycin.

In the present specification, the phrase "containing as an active ingredient" means that a therapeutically effective amount of a macrolide antimicrobial agent is contained. The term "therapeutically effective amount" as used herein means an amount of a macrolide antimicrobial agent, or an amount of a combination of a macrolide antimicrobial agent and one or more types of activators, which induces a biological or medical effect or response required by a physician, a clinician, a veterinarian, a researcher, or another appropriate expert when administered according to desirable curative measures. A preferred therapeutically effective amount is an amount that improves symptoms of a disease accompanied with destruction of bone tissue or lung tissue. In addition, the term "therapeutically effective amount" includes an amount effective for prevention, that is, an amount suitable for preventing a disease state.

Until now, the inventors have reported that when a macrolide antimicrobial agent is administered to the lung or the gingiva, the macrolide antimicrobial agent exhibits not only an antimicrobial action but also an anti-inflammatory action as well as an inhibitory action on osteoclast differentiation and bone resorption activity, by inducing DEL-1. As shown in Examples that will be described later, this time, the inventors found that by administering a macrolide antimicrobial agent, differentiation and induction of mesenchymal stem cells are achieved and regeneration of bone and lung tissues is promoted through the induction of DEL-1, thereby completing the present invention. In addition, as shown in the Examples that will be described later, regeneration of bone tissue can be promoted by administering a macrolide antimicrobial agent to an aged mouse. Accordingly, the regenerative agent for bone tissue and lung tissue of the present embodiment is suitably used for activating the bone regenerative capacity decreased due to aging or promoting the DEL-1 gene expression decreased due to aginG. That is, the regenerative agent for bone tissue and lung tissue of the present embodiment can be also considered as an activator of the bone regenerative capacity decreased due to aging or a promoter of the DEL-1 gene expression decreased due to aging.

According to the regenerative agent for bone tissue and lung tissue of the present embodiment, the regenerative agent can regenerate bone tissue or lung tissue by containing a macrolide antimicrobial agent as an active ingredient.

### [Macrolide antimicrobial agent]

The macrolide antimicrobial agent, which is an active ingredient, is a drug that inhibits bacterial protein synthesis by binding to the 50S subunit of a ribosome. Macrolide antimicrobial agents are classified according to the size of the lactone ring of an aglycone moiety. In Japan, 14-membered ring macrolide antimicrobial agents such as erythromycin (ERM), clarithromycin (CLM), and roxithromycin (RXM); and 15-membered ring macrolide antimicrobial agents such as azithromycin (AZM); and 16-membered ring macrolide antimicrobial agents such as midecamycin acetate, rokitamycin, kitasamycin, acetylspiramycin, josamycin, and midecamycin, are commercially available as pharmaceutical products.

Among them, in the regenerative agent for bone tissue and lung tissue of the present disclosure, it is preferable that the macrolide antimicrobial agent used as an active ingredient is a macrolide antimicrobial agent having a 14-membered ring or a 15-membered ring, and as shown in the Examples that will be described later, from the viewpoint that the action of increasing the expression of DEL-1 and the effect of bone regeneration are remarkable, the macrolide antimicrobial agent is more preferably one or more selected from the group consisting of erythromycin, clarithromycin, and azithromycin, and even more preferably azithromycin. These macrolide antimicrobial agents can bind particularly easily to a growth hormone secretagogue receptor (GHSR) present on the surface of DEL-1-producing cells, and therefore, it is presumed that the macrolide antimicrobial agents have an effect of further increasing the expression of DEL-1. The above-described mechanism is only speculated, and even in a case where a desired effect is obtained by a mechanism different from the above-described mechanism, the macrolide antimicrobial agents are included in the technical scope of the regenerative agent for bone tissue and lung tissue of the present disclosure.

### [Administration method]

Examples of the subject for administration include, but are not limited to, a human, a monkey, a dog, a cow, a horse, a sheep, a pig, a rabbit, a mouse, a rat, a guinea pig, and a hamster. Among them, a mammal is preferred, and a human is particularly preferred.

The administration to a human patient or an animal patient may be systemic administration or may be local administration to bone tissue or lung tissue; however, above all, from the viewpoint that proliferation of drug-resistant bacteria against the macrolide antimicrobial agent can be suppressed, and it can be made difficult to cause side effects by reducing the dosage, it is preferable that the macrolide antimicrobial agent is locally administered to bone tissue or lung tissue.

As a specific method for administration, for example, administration can be carried out intrathecal injection, intraarterial injection, intravenous injection, subcutaneous injection (including injection into the gingiva), placement at a bone-deficient site, intramuscular injection, intraarticular injection, intranasal injection, transbronchially, transpulmonarily, intramuscularly, percutaneously, or orally, by a method known to those skilled in the art.

### [Dosage]

The dosage varies depending on the body weight and age of the patient, the symptoms of the patient, the administration method, and the like; however, a person ordinarily skilled in the art can appropriately select an appropriate dosage.

Regarding the dosage of the regenerative agent for bone tissue and lung tissue of the present embodiment, it is considered appropriate to administer, in a case of oral administration, generally 100 µg or more and 1200 mg or less, preferably 200 µg or more and 1200 mg or less, and more preferably 400 µg or more and 1200 mg or less, per day in an adult (assuming a body weight of 60 kg) as an amount of the above-described macrolide antimicrobial agent, once a day or several divided doses a day.

In a case of parenteral administration, it is considered appropriate to administer, for example, in the form of an injectable preparation, generally 50 µg or more and 800 mg or less, preferably 100 µg or more and 600 mg or less, and more preferably 200 µg and 600 mg or less, per day in an adult (assuming a body weight of 60 kg) once a day or in several divided doses a day.

### <Pharmaceutical composition for regeneration of bone tissue and lung tissue>

A pharmaceutical composition for regenerating bone tissue and lung tissue of the present embodiment (hereinafter, may be simply referred to as "pharmaceutical composition of the present embodiment") contains the above-described regenerative agent for bone tissue and lung tissue and a pharmaceutically acceptable carrier.

The pharmaceutical composition of the present embodiment can regenerate bone tissue or lung tissue by containing the above-described regenerative agent for bone tissue and lung tissue as an active ingredient.

### [Pharmaceutically acceptable carrier]

As the pharmaceutically acceptable carrier, one that is conventionally used for the formulation of a pharmaceutical composition can be used without particular limitation. More specific examples thereof include binders such as gelatin, cornstarch, gum tragacanth, and gum arabic; excipients such as starch and crystalline cellulose; swelling agents such as alginic acid; solvents for an injectable preparation, such as water, ethanol, and glycerin; and tacky adhesives such as a rubber-based tacky adhesive and a silicone-based tacky adhesive.

The pharmaceutical composition of the present embodiment may further include additives. Examples of the additives include lubricating agents such as calcium stearate and magnesium stearate; sweeteners such as sucrose, lactose, saccharin, and maltitol; flavoring agents such as peppermint and Akamono (Japanese azalea) oil; stabilizers such as benzyl alcohol and phenol; buffer agents such as a phosphoric acid salts and sodium acetate; dissolution aids such as benzyl benzoate and benzyl alcohol; antioxidants; and preservatives.

The pharmaceutical composition of the present embodiment can be formulated by suitably combining the above-described regenerative agent for bone tissue and lung tissue with the above-described pharmaceutically acceptable carrier and additives and mixing the mixture in the form of unit dosage required for generally accepted pharmaceutical practice.

The pharmaceutical composition of the present embodiment may be in a dosage form that is used orally or may be in a dosage form that is used parenterally; however, a dosage form that is used parenterally is preferred. Examples of the dosage form that is used orally include a tablet, a capsule, an elixir, and a microcapsule. Examples of the dosage form that is used parenterally include an injectable preparation, a nebula, a suppository, and a patch.

The pharmaceutical composition of the present embodiment may be used in combination with a therapeutic agent for another disease. For example, by using the above-described regenerative agent for bone tissue and lung tissue in combination with an anti-inflammatory agent, an antimicrobial agent other than a macrolide antimicrobial agent, hyaluronic acid used for treating cartilage, and the like, bone tissue or lung tissue can be regenerated while reducing the symptoms of the target disease.

The above-described regenerative agent for bone tissue and lung tissue and other drugs may be formulated into the same preparation or may be formulated into different preparations. **In** addition, each of the preparations may be administered by the same administration route or may be administered by different administration routes. **In** addition, each of the preparations may be administered simultaneously, may be administered sequentially, or may be administered separately with an interval of a certain time or period. According to an embodiment, the above-described regenerative agent for bone tissue and lung tissue and other drugs may be prepared into a kit including them.

The pharmaceutical composition of the present embodiment is preferably used for treating a disease accompanied with the destruction of bone tissue or lung tissue. Preferred examples of the disease accompanied with the destruction of bone tissue or lung tissue include periodontal diseases, osteoporosis, rheumatoid arthritis, pulmonary fibrosis, bacterial pneumonia (including aspiration pneumonia), interstitial pneumonia, and pulmonary emphysema.

In addition, as shown in the Examples that will be described later, regeneration of bone tissue can be promoted by administering a macrolide antimicrobial agent to an aged mouse. Therefore, the pharmaceutical composition of the present embodiment is preferably used for treating or preventing deterioration of bone regenerative capacity associated with aging.

According to an embodiment, the present invention provides a use application of the above-described macrolide antimicrobial agent for producing a pharmaceutical composition for regenerating bone tissue and lung tissue. Here, examples of the above-described macrolide antimicrobial agent include agents similar to those described above.

### [Examples]

Hereinafter, the present invention will be described by way of Examples; however, the present invention is not intended to be limited to the following Examples.

### [Example 1]

### (DEL-1-dependent bone formation promoting action by erythromycin as macrolide antimicrobial agent)

Experimental periodontitis model mice in which teeth are ligated with silk threads are useful models for inflammatory bone destruction research because inflammations and bone resorption occur in about 10 days after the ligation. The above-described experimental periodontitis models were produced by using 6-week-old wild-type mice (N = 8, male C57BL/6N mice, manufactured by CLEA Japan, Inc.), 22-month-old aging mice (N = 4, male C57BL/6N mice, manufactured by CLEA Japan, Inc.), and 6-week-old DEL-1-deficient mice (N = 8, male C57BL/6N mice (manufactured by CLEA Japan, Inc.), a method for producing the deficient mice was as described in Non Patent Document 1), the silk threads were removed 10 days after the ligation, a remission phase of inflammation of 5 days passed, and in the wild-type mice, new bone formation was recognized at the site of resorbed bone defect while no repair mechanism of the periodontal tissue by DEL-1 was recognized.

Thus, when these aging mice and DEL-1-deficient mice were inoculated with 1 µg of recombinant DEL-1 into the gingival tissue, bone repair similar to that of the 6-week-old wild-type mice occurred.

Next, from the cranial bones of 3-day-old wild-type mice (N = 6, male C57BL/6N mice, manufactured by CLEA Japan, Inc.) and 3-day-old DEL-1-deficient mice (N = 6, male C57BL/6N mice (manufactured by CLEA Japan, Inc.), a method for producing the deficient mice was as described in Non Patent Document 1), osteoblasts were isolated by allowing 0.1% by mass of type 1 collagenase and 0.2% by mass of dispase to act at 37°C for 20 minutes, and the cells were cultured in α-MEM and a bone differentiation medium (containing 50 µg/ml ascorbic acid and 10 mM β-glycerol 2-phosphate). Ethanol (final concentration 0.1% by mass) and erythromycin (10 µg/mL) were added into the medium in which the osteoblasts (1 × 10⁴ cells/mL) derived from the wild-type mice or the DEL-1-deficient mice were cultured. The medium was exchanged every 3 days, and after 15 days, the cells were stained with Alizarin red S, followed by performing of bone nodule measurement.

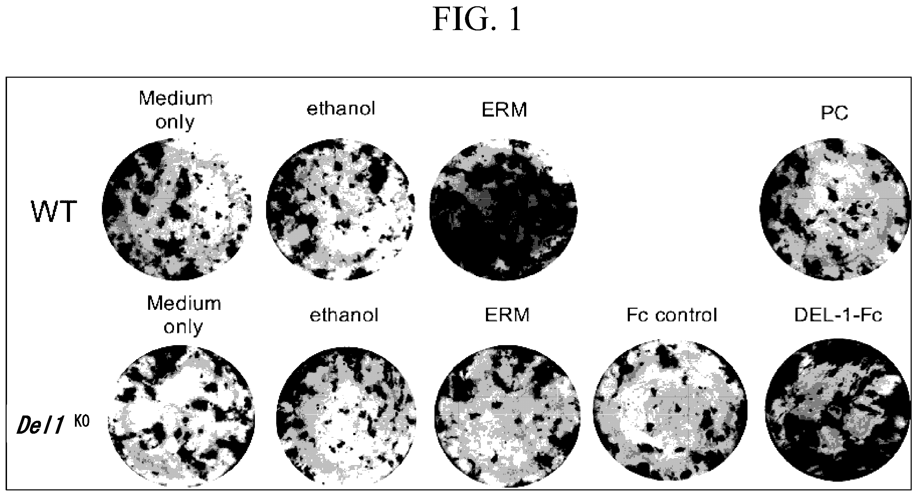

The results are shown in FIG. 1. In FIG. 1, "ERM" denotes an erythromycin-added group, "Fc control" denotes a recombinant DEL-1 control-added group, "PC" denotes a penicillin-added group, and DEL-1-Fc denotes a recombinant DEL-1-added group.

As shown in FIG. 1, the formation of bone nodules was recognized in the erythromycin-added group of osteoblasts derived from wild-type mice and in the DEL-1-Fc-added group of osteoblasts derived from DEL-1-deficient mice. On the other hand, the formation of bone nodules was not recognized in the erythromycin-added group of osteoblasts derived from DEL-1-deficient mice that did not produce DEL-1.

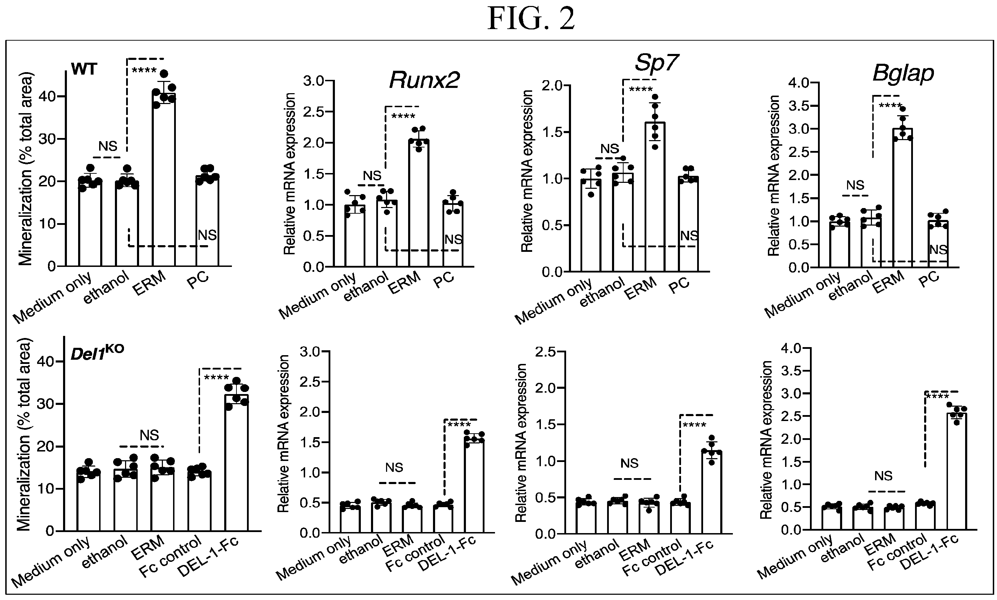

Next, the proportion (%) of bone nodules with respect to the observation region in the above-described osteoblasts was calculated from microscopic images by using image processing software (NIH Image). In addition, RNA was extracted from each of the osteoblasts by using an RNeasy Kit (manufactured by QIAGEN NV), subsequently cDNA was synthesized by using a cDNA synthesis kit (manufactured by Thermo Fisher Scientific, Inc.), and the relative expression levels of RUNX2 mRNA, Sp7 mRNA, and Bglap mRNA, which are important for bone regeneration, were checked by an RT-PCR method (Quantstudio3, Thermo Fisher Scientific, Inc.). Regarding primers, designed primers manufactured by Thermo Fisher Scientific, Inc. were used. The Assay IDs of the primers used are shown below. The results are shown in FIG. 2.

### (Assay ID of each primer)

Edil3 (Mm01291247_m1)
Runx2 (Mm00501584_m1)
Bglap (Mm03413826_mH)
Sp7 (Mm00504574_m1)
Gapdh (expression control) (Mm99999915_g1)

As shown in FIG. 2, a significant increases in the proportion of bone nodules was recognized in the erythromycin-added group of osteoblasts derived from the wild-type mice and in the DEL-1-Fc-added group of osteoblasts derived from the DEL-1-deficient mice. On the other hand, an increase in the proportion of bone nodules was not recognized in the erythromycin-added group of osteoblasts derived from the DEL-1-deficient mice. In addition, increases in the expression of RUNX2 mRNA, Sp7 mRNA, and Bglap mRNA, which are important for bone regeneration, were recognized in the erythromycin-added group of osteoblasts derived from the wild-type mice and in the DEL-1-Fc-added group of osteoblasts derived from the DEL-1-deficient mice.

### [Example 2]

### (Bone regeneration-inducing effect by erythromycin as macrolide antimicrobial agent)

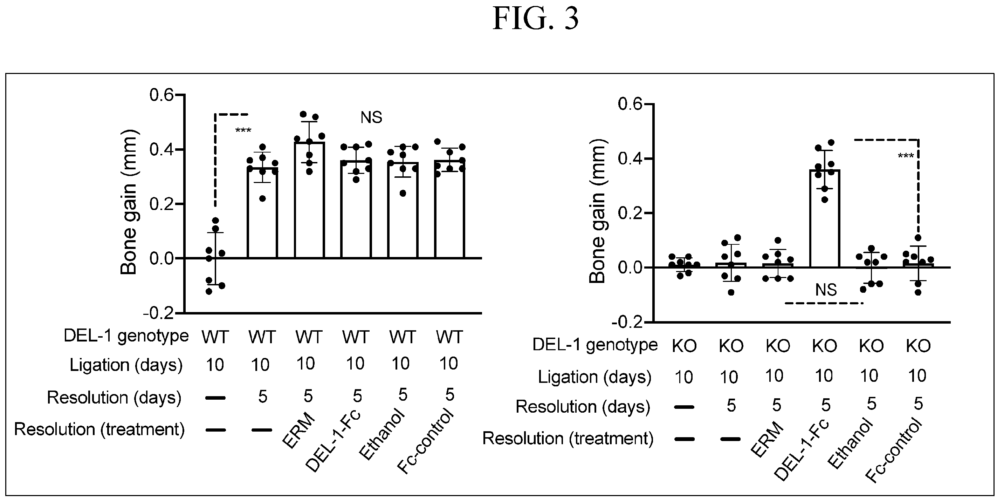

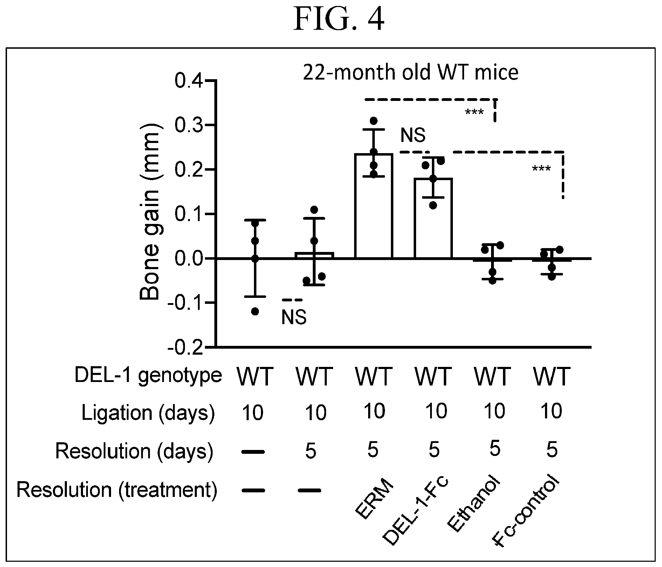

The experimental periodontitis models described in Example 1 were produced by using 6-week-old wild-type mice (N = 8, male C57BL/6N mice, manufactured by CLEA Japan, Inc.), 22-month-old aging mice (N = 4, male C57BL/6N mice, manufactured by CLEA Japan, Inc.), and 6-week-old DEL-1-deficient mice (N = 8, male C57BL/6N mice (manufactured by CLEA Japan, Inc.), a method for producing the deficient mice was as described in Non Patent Document 1), the silk threads were removed 10 days after the ligation to experimentally induce periodontitis, subsequently a bone regeneration period of 5 days was provided, and then the amount of regeneration of resorbed bone was measured using a stereomicroscope and image measurement software (manufactured by Nikon Corporation). Erythromycin (10 mg/kg) and ethanol (final concentration 0.1% by mass) were intraperitoneally administered once after the removal of the silk threads. In addition, DEL-1 (1 µg) and Fc control (0.84 µg) were directly inoculated into the gingiva after the removal of the silk threads. The results are shown in FIG. 3 (left: wild-type mice, right: DEL-1-deficient mice) and FIG. 4 (aging mice).

As shown in FIG. 3, in the wild-type mice, bone regeneration was recognized irrespective of the presence or absence of the administration of erythromycin. On the other hand, in the DEL-1-deficient mice, bone regeneration was not recognized except for the group inoculated with DEL-1.

As shown in FIG. 4, in the aging mice, bone regeneration was not recognized even in a case where a bone regeneration period of 5 days was provided; however, bone regeneration was recognized as a result of the administration of erythromycin or the inoculation with DEL-1.

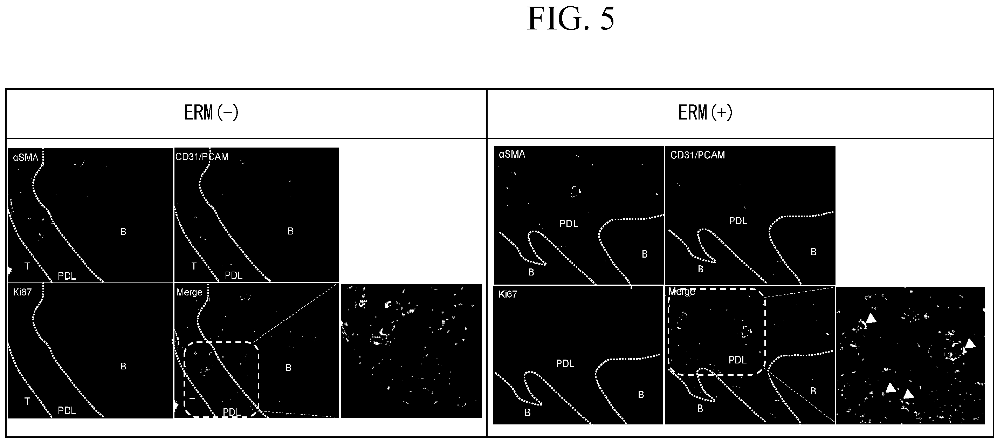

Next, jawbones of the wild-type mice to which erythromycin had or had not been administered were extracted and fixed, and then slice preparations were produced. Next, immunofluorescence staining was performed by using an anti-α-SMA antibody (manufactured by Abcam, Ltd.) fluorescently labeled with Alexa Fluor (registered trademark) 488 (manufactured by Thermo Fisher Scientific, Inc.), an anti-CD31/PECAM-1 antibody (manufactured by Thermo Fisher Scientific, Inc.) fluorescently labeled with Alexa Fluor (registered trademark) 647 (manufactured by Thermo Fisher Scientific, Inc.), and an anti-Ki67 antibody (manufactured by R&D Systems, Inc.) fluorescently labeled with Alexa Fluor (registered trademark) 594 (manufactured by Thermo Fisher Scientific, Inc.). Observation images (magnification: 200 times) obtained with a fluorescence microscope (ECLIPSE Ni-E, manufactured by Nikon Corporation) are shown in FIG. 5.

As shown in FIG. 5, it was revealed that the proportion of α-SMA-positive mesenchymal stem cells was increased by the administration of erythromycin. That is, it was revealed that the administration of erythromycin promotes the proliferation and differentiation of the mesenchymal stem cells present in the periodontal membrane and provides an environment in which the bone and the surrounding tissue are easily regenerated.

### [Example 3]

### (Bone regenerative action by erythromycin in rheumatoid arthritis model)

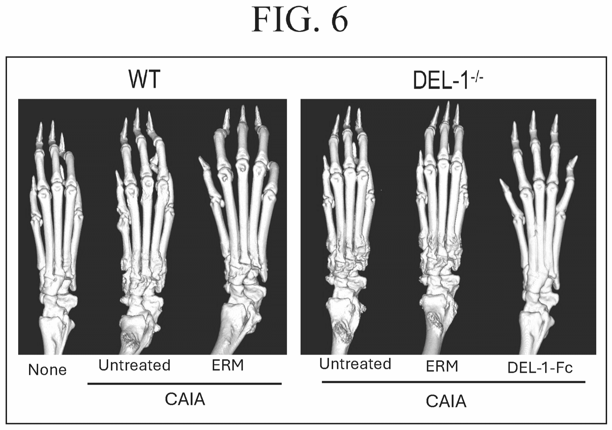

Rheumatoid arthritis (CAIA) (a collagen antibody-administered arthritis-inducing cocktail manufactured by Chondrex, Inc. was used, and the details of the production method were performed according to the protocol provided by Iwai Chemicals Co., Ltd.) was induced by using 6-week-old wild-type mice (N = 4, male C57BL/6N mice, manufactured by CLEA Japan, Inc.) and 6-week-old DEL-1-deficient mice (N = 4, male C57BL/6N mice (manufactured by CLEA Japan, Inc.), the method for producing the deficient mice was as described in Non Patent Document 1), and erythromycin (10 mg/kg) was intraperitoneally administered once every 3 days from the 1st day after the induction. Regarding the DEL-1-deficient mice, a group in which DEL-1 (10 µg) was administered through the tail vein once every 3 days from the 1st day after the CAIA induction, was also prepared. Nine days after the administration, the hind limb of each mouse was examined by microCT. The results are shown in FIG. 6.

As shown in FIG. 6, in the wild-type rheumatoid arthritis model mice, bone destruction was suppressed by the administration of erythromycin. On the other hand, in the DEL-1-deficient rheumatoid arthritis model mice, bone destruction was not suppressed by the administration of erythromycin; however, bone destruction was suppressed by the administration of DEL-1.

### [Example 4]

### (Lung tissue regenerative action by erythromycin for acute lung disorder)

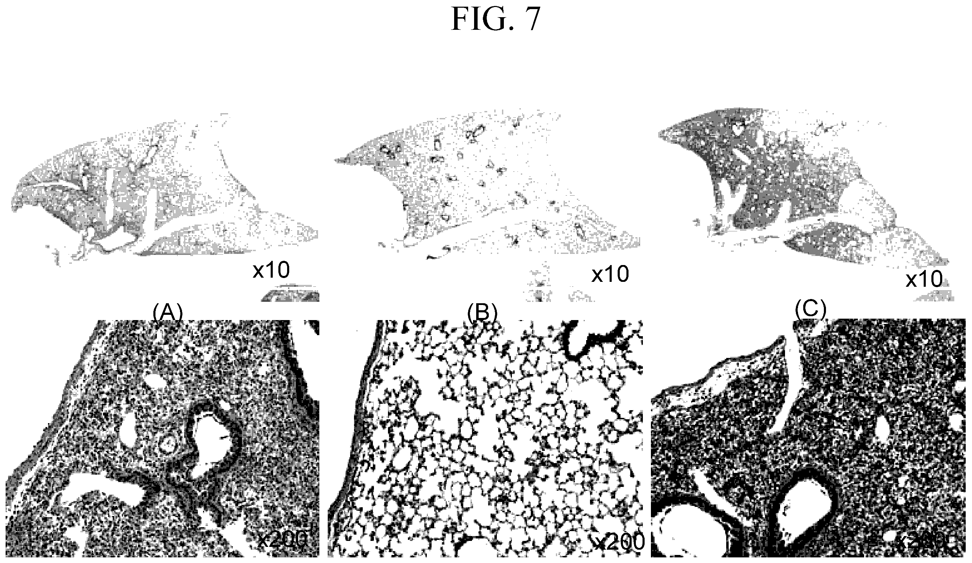

Acute lung disorder was induced by administering lipopolysaccharide (LPS) to the tracheae and bronchi of mice (the details of a production method were as described in Non Patent Document 1). Erythromycin (10 mg/kg) was administered to 6-week-old wild-type mice (N = 4, male C57BL/6N mice, manufactured by CLEA Japan, Inc.) and 6-week-old DEL-1-deficient mice (N = 4, male C57BL/6N mice (manufactured by CLEA Japan, Inc.), a method for producing the deficient mice was as described in Non-Patent Document 1), by intraperitoneal administration once a day for a total of two weeks. After the administration, the lungs were extracted from each mouse and fixed, and then slice preparations were produced. Next, hematoxylin and eosin (HE) staining was performed. Observation images (magnifications: 10 times and 200 times) obtained with a microscope (manufactured by Nikon Corporation, ECLIPSE Ni-E) are shown in FIG. 7. In FIG. 7, (A) shows the lung tissue of a mouse to which erythromycin was not administered, (B) shows the lung tissue of a wild-type mouse to which erythromycin was administered, and (C) shows the lung tissue of a DEL-1-deficient mouse to which erythromycin was administered.

As shown in FIG. 7(A), in the wild-type mouse to which erythromycin was not administered, infiltration of neutrophils and tissue destruction in a wide range were recognized, and furthermore, since the lung tissue was fibrosed, the alveolar structure was destroyed.

As shown in FIG. 7(B), in the wild-type mouse to which erythromycin was administered, normal tissue repair and restructuring of the alveolar structure were recognized. On the other hand, as shown in FiG. 7(C), in the DEL-1-deficient mouse to which erythromycin was administered, normal tissue repair and restructuring of the alveolar structure were not recognized.

From these, it became clear that the tissue repair and restructuring of the alveolar structure by erythromycin are DEL-1-dependent.

### [Example 5]

### (DEL-1-inducing action and bone regenerative action by various macrolide antimicrobial agents)

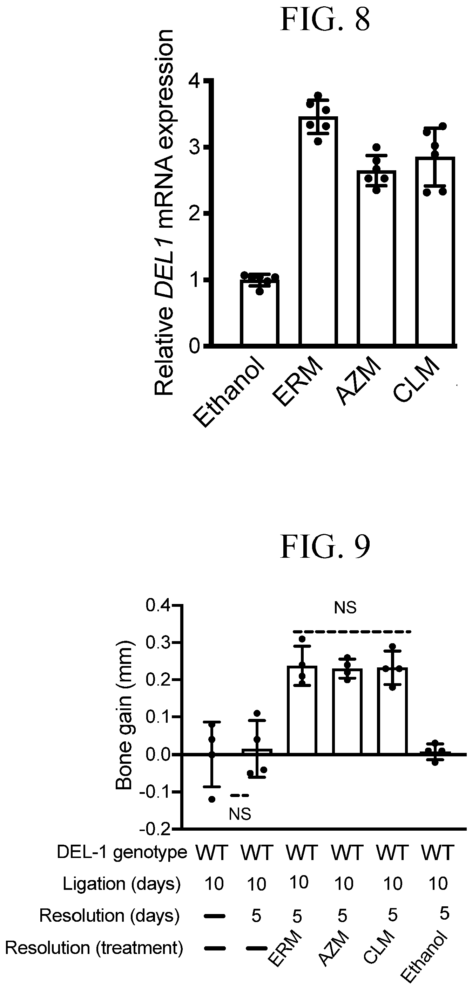

Erythromycin (ERM) (10 µg/mL), azithromycin (AZM) (10 µg/mL), or clarithromycin (CLM) (10 µg/mL), all of which are macrolide antimicrobial agents, was added to vascular endothelial cells (1 × 10⁵ cells/mL) that produce DEL-1. After 4 hours from the addition, cDNA was synthesized from each of the vascular endothelial cells by using a cDNA synthesis kit (manufactured by Thermo Fisher Scientific, Inc.), and the relative expression level of DEL-1 mRNA was checked by an RT-PCR method (Quantstudio3, manufactured by Thermo Fisher Scientific, Inc.). Regarding primers, designed primers manufactured by Thermo Fisher Scientific, Inc. were used. The Assay IDs of the primers used are shown below. The results are shown in FIG. 8.

### (Assay ID of each primer)

Edil3 (Mm01291247_m1)
Gapdh (expression control) (Mm99999915_g1)

An increase in the expression of DEL-1 mRNA was recognized as a result of the addition of each of the macrolide antimicrobial agents. In addition, there was no difference in the expression level of DEL-1 mRNA between the groups to which the macrolide antimicrobial agents were added.

Next, the experimental periodontitis model described in Example 1 was produced by using 22-month-old aging mice (N = 4, male C57BL/6N mouse, manufactured by CLEA Japan, Inc.), the silk threads were removed 10 days after the ligation to experimentally induce periodontitis, subsequently a bone regeneration period of 5 days was provided, and the amount of regeneration of resorbed bone was measured using a stereomicroscope and image measurement software (manufactured by Nikon Corporation). Erythromycin (ERM) (20 mg/kg), azithromycin (AZM) (20 mg/kg), clarithromycin (CLM) (20 mg/kg), and ethanol (final concentration 0.1% by mass) as a control were intraperitoneally administered once after the removal of the silk threads. The results are shown in FIG. 9.

As shown in FIG. 9, in the aging mice to which no macrolide antimicrobial agent was administered, bone regeneration was not recognized even in a case where a bone regeneration period of 5 days was provided; however, bone regeneration was recognized in the aging mice to which the macrolide antimicrobial agents (ERM, AZM, and CLM) were administered. In addition, there was no difference in the amount of bone regeneration between the macrolide antimicrobial agent-administered groups.

From the above, it was revealed that bone tissue and lung tissue can be effectively regenerated by administering a macrolide antimicrobial agent, through the induction of DEL-1.

### [Example 6]

### (Bone regeneration-inducing effect 1 by various macrolide antimicrobial agents)

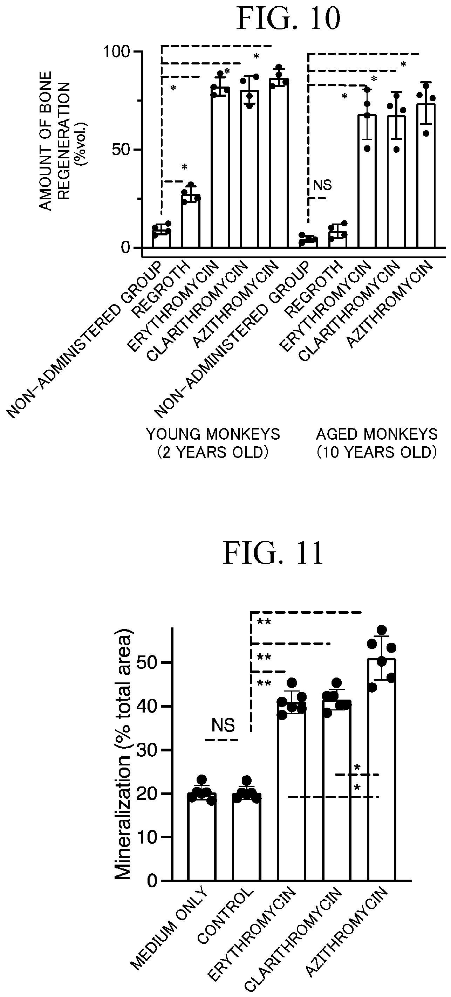

Bone defects with a diameter of 2 cm and a depth of 2 cm were artificially produced in the jawbones of young (2 years old) and aged (10 years old) cynomolgus monkeys by using a round steel bar. Next, 200 µg of each of erythromycin, clarithromycin, and azithromycin were added to the bone defect sites. As controls, a non-administered group and a group administered with 200 µg of REGROTH (registered trademark), which has been approved as a bone regenerative agent, were also prepared. The amount of bone regeneration after one month from the administration of each drug was checked by using microCT. The results are shown in FIG. 10. In FIG. 10, the symbol * indicates p < 0.01.

As shown in FIG. 10, in the erythromycin-, clarithromycin-, and azithromycin-administered groups, a bone regeneration effect was recognized at a dosage in the order of µg in both young and aged cynomolgus monkeys. On the other hand, with REGROTH, a bone regeneration effect (bone regeneration effect expected in a case of being used in clinical practice of a periodontal disease) as expected (about 30%) was recognized in the young cynomolgus monkeys; however, a sufficient bone regeneration effect was not recognized at a dosage in the order of µg in the aged cynomolgus monkeys.

### [Example 7]

### (Bone regeneration-inducing effect 2 by various macrolide antimicrobial agents)

From the cranial bones of 3-day-old wild-type mice (N = 6, male C57BL/6N mice, manufactured by CLEA Japan, Inc.), osteoblasts were isolated by allowing 0.1% by mass of type 1 collagenase and 0.2% by mass of dispase to act at 37°C for 20 minutes, and the cells were cultured in α-MEM and a bone differentiation medium (containing 50 µg/ml ascorbic acid and 10 mM β-glycerol 2-phosphate). Ethanol (final concentration 0.1% by mass), or erythromycin, clarithromycin, or azithromycin (10 µg/mL each) was added into the medium in which the osteoblasts (1 × 10⁴ cells/mL) derived from the wild-type mice were cultured. The medium was exchanged every 3 days, and after 15 days, the cells were stained with Alizarin red S, followed by performing of bone nodule measurement. The results are shown in FIG. 11. In FIG. 11, the symbol * indicates p < 0.05, and the symbol ** indicates p < 0.01. In addition, the control is an ethanol (final concentration 0.1% by mass)-added group.

As shown in FIG. 11, the formation of bone nodules was recognized as a result of the addition of erythromycin, clarithromycin, and azithromycin, and the formation of bone nodules by the addition of azithromycin was particularly favorable.

### [Example 8]

### (Bone regeneration-inducing effect 1 in aged mice by various macrolide antimicrobial agents)

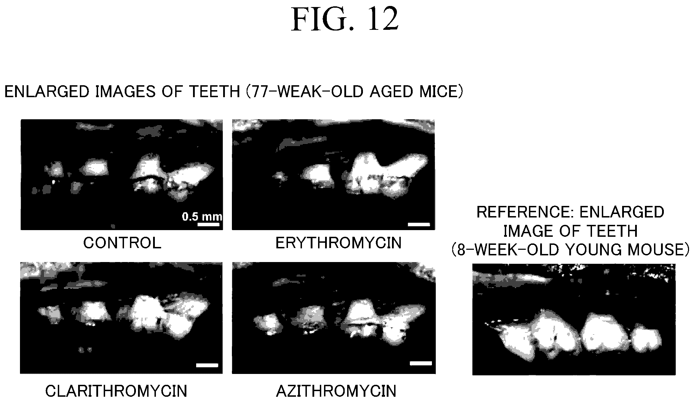

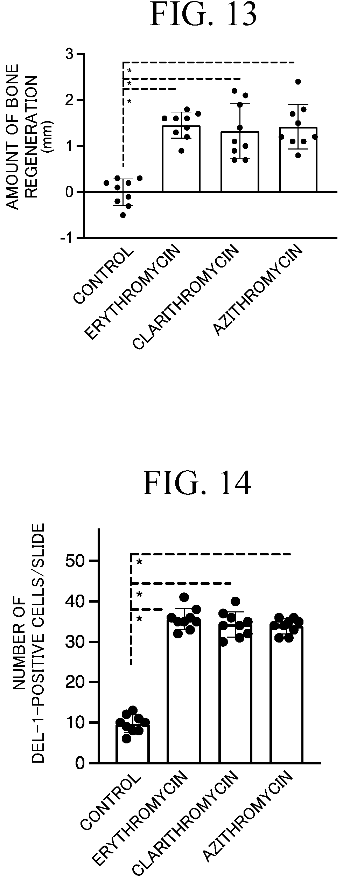

A macrolide antimicrobial agent (erythromycin, clarithromycin, or azithromycin) was intraperitoneally administered to 77-week-old wild-type mice (N = 9, male C57BL/6N mice, manufactured by CLEA Japan, Inc.) at 10 mg/kg once a day for 7 days. The amount of teeth regeneration was measured with a stereomicroscope and image measurement software (manufactured by Nikon Corporation). The results are shown in FIG. 12 (stereomicroscopic images) and FIG. 13 (amount of bone regeneration). In FIG. 12 and FIG. 13, the control is a group to which no macrolide antimicrobial agent was administered. In addition, in FIG. 13, the symbol * indicates p < 0.05.

As shown in FIG. 12, the young (8 weeks old) mice for reference had proper tooth shapes, whereas in the aged (77 weeks old) mice (control group), the teeth were wearing down, and bone resorption was also significant. In contrast, as shown in FIG. 12 and FIG. 13, in the macrolide antimicrobial agent-administered groups, the bone regenerative capacity was activated even in the aged mice, and bone regeneration was recognized.

### [Example 9]

### (Bone regeneration-inducing effect 2 in aged mice by various macrolide antimicrobial agents)

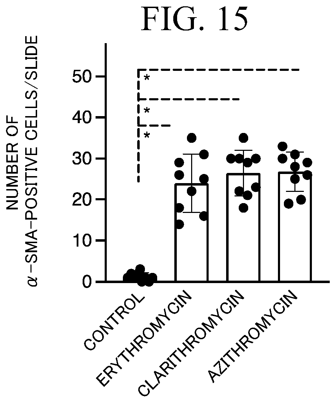

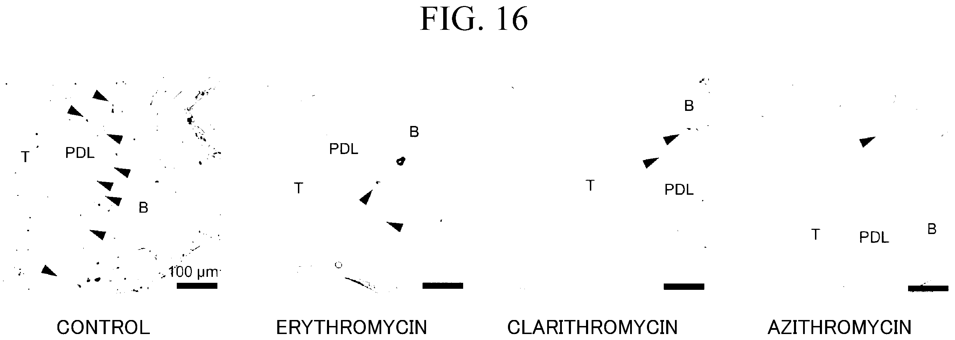

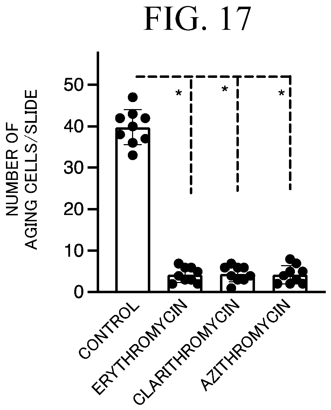

A macrolide antimicrobial agent (erythromycin, clarithromycin, or azithromycin) was intraperitoneally administered to 77-week-old wild-type mice (N = 9, male C57BL/6N mice, manufactured by CLEA Japan, Inc.) at 10 mg/kg once a day for 7 days. Next, immunofluorescence staining was performed by using an anti-DEL-1 antibody (manufactured by Abcam, Ltd.) and an anti-α-SMA antibody (manufactured by Abcam, Ltd.), both of which were fluorescently labeled with Alexa Fluor (registered trademark) 488 (manufactured by Thermo Fisher Scientific, Inc.), and an anti-CD31/PECAM-1 antibody (manufactured by Thermo Fisher Scientific, Inc.) fluorescently labeled with Alexa Fluor (registered trademark) 594 (manufactured by Thermo Fisher Scientific, Inc.). In addition, aging cells were stained by using an aging cell detection kit (manufactured by Fujifilm Corporation). Observation (magnification: 200 times) with a fluorescence microscope (manufactured by Nikon Corporation, ECLIPSE Ni-E) was performed, and the numbers of cells of DEL-1-positive cells, α-SMA-positive cells, and aging cells on a randomly picked-up slide were measured. The results are shown in FIG. 14 (graph for DEL-1-positive cells), FIG. 15 (graph for α-SMA-positive cells), FIG. 16 (observation image of aging cells), and FIG. 17 (graph for aging cells).

As shown in FIG. 14, an increase in the number of the DEL-1-positive cells was observed in the macrolide antimicrobial agent-administered group. In addition, although an observation image is not shown, an increase in the cells expressing DEL-1 was strongly recognized in the vicinity of CD31-positive cells of the periodontal membrane (PDL), which is a space that connects teeth and bones important for regeneration, as a result of the administration of macrolide antimicrobial agents.

As shown in FIG. 15, proliferation of α-SMA-positive mesenchymal stem cells capable of differentiating into osteoblasts that make bones in the periodontal membrane was recognized as a result of administration of the macrolide antimicrobial agents.

As shown in FIG. 16 and FIG. 17, it became clear that there is not only a bone induction effect but also a senoritic effect capable of removing aging cells. Since DEL-1 induced by various macrolide antimicrobial agents exhibits these effects, activation of the bone regenerative capacity in the aged with deteriorated bone regenerative capacity can be expected.

### [Example 10]

### (Bone regeneration-inducing effect in bone cells obtained by differentiating mesenchymal stem cells derived from iPS cells, due to various macrolide antimicrobial agents)

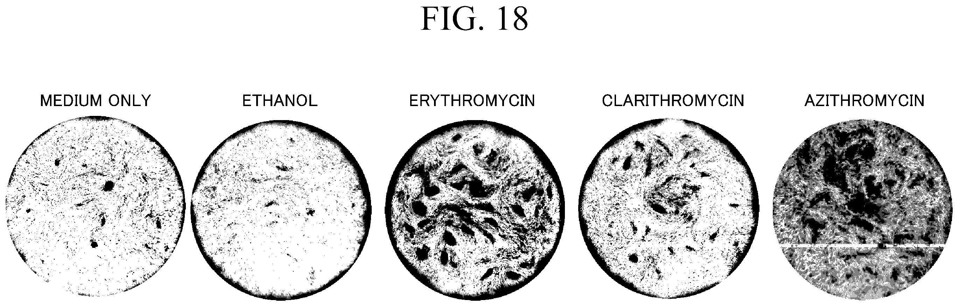

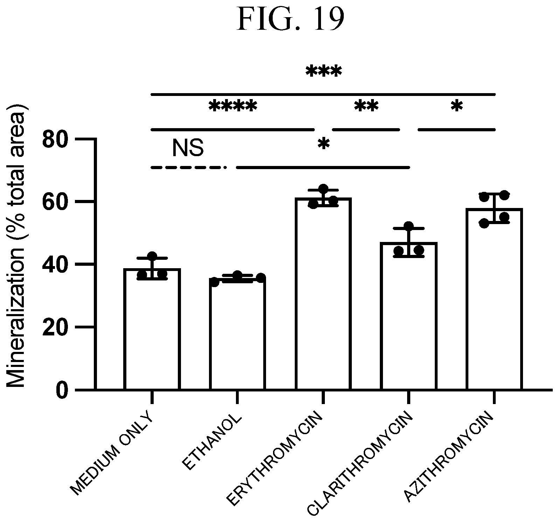

iCell (registered trademark) mesenchymal stem cells (iCell (registered trademark) MSCs) (#01279, manufactured by Fujifilm Corporation) (1 × 10⁴ cells/mL) obtained by inducing human mesenchymal stem cells from iPS cells, were cultured in α-MEM and a bone differentiation medium (containing 50 µg/mL ascorbic acid and 10 mM β-glycerol 2-phosphate). Ethanol (final concentration 0.1% by mass), or erythromycin, clarithromycin, or azithromycin (10 µg/mL for each) was added to the medium. The medium was exchanged every 3 days, and after 15 days, the cells were stained with Alizarin red S, followed by performing of bone nodule measurement. The results are shown in FIG. 18 (stained images) and FIG. 19 (graph showing the proportion of bone nodules). In FIG. 18 and FIG. 19, the ethanol (final concentration 0.1% by mass)-added group is a control. In FIG. 19, the symbol * indicates p ≤ 0.05, the symbol ** indicates p ≤ 0.01, the symbol *** indicates p ≤ 0.001, and the symbol **** indicates p ≤ 0.0001.

As shown in FIG. 18 and FIG. 19, the addition of erythromycin, clarithromycin, and azithromycin promoted the differentiation of mesenchymal stem cells induced from human iPS into bone cells, and the formation of bone nodules was recognized.

### [Industrial Applicability]

According to the regenerative agent for bone tissue and lung tissue and the pharmaceutical composition for regenerating bone tissue and lung tissue of the present disclosure, the bone tissue or the lung tissue can be regenerated, and a disease accompanied with the destruction of the bone tissue or the lung tissue can be effectively treated.

## Claims

1. A macrolide antimicrobial agent for use in regenerating bone tissue in a patient, wherein the macrolide antimicrobial agent is azithromycin.

2. The macrolide antimicrobial agent for use in regenerating bone tissue according to Claim 1, wherein the macrolide antimicrobial agent is parenterally administered to bone tissue in the form of an injectable preparation, and wherein the dose is 50 µg to 200 µg per day in an adult.

3. The macrolide antimicrobial agent for use in regenerating bone tissue according to Claim 1, wherein the macrolide antimicrobial agent is administered orally, and the dose is 100 µg to 1200 mg per day in an adult.

4. A macrolide antimicrobial agent for use in treating a disease accompanied with the destruction of bone tissue, wherein the macrolide antimicrobial agent is azithromycin , and
wherein the disease accompanied with the destruction of bone tissue is osteoporosis or rheumatoid arthritis.

## Patentansprüche

1. Makrolidantibiotikum zur Verwendung bei der Regenerierung von Knochengewebe bei einem Patienten, wobei das Makrolidantibiotikum Azithromycin ist.

2. Makrolidantibiotikum zur Verwendung bei der Regenerierung von Knochengewebe nach Anspruch 1, wobei das Makrolidantibiotikum parenteral an Knochengewebe in der Form eines injizierbaren Präparats verabreicht wird und wobei die Dosis 50 µg bis 200 µg pro Tag bei einem Erwachsenen beträgt.

3. Makrolidantibiotikum zur Verwendung bei der Regenerierung von Knochengewebe nach Anspruch 1, wobei das Makrolidantibiotikum oral verabreicht wird und die Dosis 100 µg bis 1200 µg pro Tag bei einem Erwachsenen beträgt.

4. Makrolidantibiotikum zur Verwendung bei der Behandlung einer Erkrankung, die von der Zerstörung von Knochengewebe begleitet ist, wobei das Makrolidantibiotikum Azithromycin ist und
wobei die Erkrankung, die von der Zerstörung von Knochengewebe begleitet ist, Osteoporose oder rheumatoide Arthritis ist.

## Revendications

1. Agent antimicrobien de type macrolide pour utilisation dans la régénération de tissu osseux chez un patient, où l'agent antimicrobien de type macrolide est de l'azithromycine.

2. Agent antimicrobien de type macrolide pour utilisation dans la régénération de tissu osseux selon la revendication 1, où l'agent antimicrobien de type macrolide est administré par voie parentérale dans le tissu osseux sous forme de préparation injectable, et où la dose est d'entre 50 µg et 200 µg par jour chez un adulte.

3. Agent antimicrobien de type macrolide pour utilisation dans la régénération de tissu osseux selon la revendication 1, où l'agent antimicrobien de type macrolide est administré par voie orale, et la dose est d'entre 100 µg et 1200 mg par jour chez un adulte.

4. Agent antimicrobien de type macrolide pour utilisation dans le traitement d'une maladie accompagnée de la destruction de tissu osseux, où l'agent antimicrobien de type macrolide est de l'azithromycine, et
où la maladie accompagnée de la destruction de tissu osseux est l'ostéoporose ou la polyarthrite rhumatoïde.
